Europäisches Patentamt

(19) European Patent Office

Office européen des brevets

(11) Publication number: **0 220 825**
**B1**

(12) **EUROPEAN PATENT SPECIFICATION**

(45) Date of publication of the patent specification:
06.09.89

(51) Int. Cl.⁴: **A61K 31/455, A61K 47/00**

(21) Application number: 86307195.7

(22) Date of filing: 18.09.86

(54) Pharmaceutical composition for topical application.

(30) Priority: 18.09.85 GB 8523036

(43) Date of publication of application:
06.05.87 Bulletin 87/19

(45) Publication of the grant of the patent:
06.09.89 Bulletin 89/36

(84) Designated Contracting States:
AT BE CH DE FR IT LI LU NL SE

(56) References cited:
GB-A- 964 444
US-A- 2 431 558

CHEMICAL ABSTRACTS, vol. 99, no. 7, 15th
August 1983, page 15, abstract no. 47531h, Columbus,
Ohio, US; W.J. ALBERY et al.: "Percutaneous
absorption: transport in the dermis", & INT. J.
PHARM. 1983, 15(2), 125-48
ARZNEIMITTEL-FORSCHUNG, vol. 11, no. 2,
January 1961, pages 102-104, Aulendorf, DE; D.
BRAASCH et al.: "Untersuchungen der menschlichen
Hautdurchblutung mit einem neuen Wärmeleitmesser
bei lokaler Einwirkung von Nicotinsäureestern"

(73) Proprietor: **DIOMED DEVELOPMENTS LIMITED, Tatmore
Place, Gosmore Nr. Hitchin, Hertfordshire,
SG4 7QR(GB)**

(72) Inventor: **Whitefield, Martin, 19, Parkway, London,
NW11(GB)**

(74) Representative: **Collier, Jeremy Austin Grey et al,
J.A.Kemp & Co. 14, South Square Gray's Inn, London
WC1R 5EU(GB)**

ACTORUM AG

**Description.**

This invention relates to pharmaceutical compositions for topical application, and provides a novel composition useful for preventing bed sores.

Patients who are unable for any reason to move themselves frequently suffer from so called "bed sores", more properly pressure sores, which are local areas of ulceration caused by prolonged pressure preventing an adequate blood supply to that area.

It is known to use nicotinate esters, e.g. methyl nicotinate, in compositions for topical application to the skin to produce pronounced local erythema. In such compositions, the nicotinate is normally used in a concentration of, for example, 1 to 2.5%. Such erythema causes a local sensation of warmth which is useful in alleviating certain types of local pain usually of muscular origin. The generation of pronounced and perhaps persistent erythema in an area prone to a pressure sore would, however, be inappropriate since that would tend to exacerbate the problem.

It has now unexpectedly been discovered that hexyl nicotinate can be used to improve local blood supply to the skin without causing obvious erythema. This has been demonstrated by the tests described below. The consequence of this discovery is that it is possible to produce a composition which may be used to increase blood flow in areas affected by pressure sores without inducing local irritation. The local restriction in blood flow caused by the pressure is thereby alleviated and this substantially prevents the formation of a pressure sore.

The present invention accordingly provides a pharmaceutical composition for topical application to skin areas prone to pressure sores, comprising hexyl nicotinate in a concentration from 0.025 to 0.075% by weight based on the weight of the composition, i.e. a concentration sufficient to improve local blood flow (i.e. increased blood cell flux) in an epidermal area without causing obvious erythema, associated with an appropriate vehicle.

The hexyl nicotinate ester is "slow-acting", i.e. it does not produce a too rapid and transient effect as it is desirable to reduce the frequency of application of the composition to avoid the inconvenience associated with frequent application. Rapidity of action is related to speed of absorption which depends on molecular size and oil/water solubility. Smaller molecules and those with an oil : water partition coefficient of about 1:1 are absorbed most efficiently. In order to slow down the rate of absorption it is necessary to increase the size of the molecule, and this may alter the solubility characteristics of the molecule in one of two ways, each of which can slow down penetration for the following reasons:

(1) If the molecule becomes more lipid soluble, a depot of the ester is formed in the stratum corneum from which it is only slowly released into the more aqueous environment of the viable epidermis;

(2) If the molecule becomes more water soluble, the ester tends to remain longer in the formulation and is only slowly released into the stratum corneum.

Methyl nicotinate, for example, is a small molecule with an oil : water partition coefficient of about 1:1. It therefore acts very rapidly and is unsuitable for use in the present invention. Hexyl nicotinate is an appropriate ester with more lipid and less water solubility.

As already mentioned, known nicotinate-containing compositions often contain 1% or more of a nicotinate ester. This is substantially more than the proportion required in the present invention. The appropriate concentration range for hexyl nicotinate is 0.025 to 0.075% by weight of the composition. Concentrations in the range 0.1 to 0.2% cause erythema in a significant proportion of patients.

The composition is preferably made up in the form of a lotion for ease of application. For this purpose the vehicle for the hexyl nicotinate may be a combination of stearic acid in a proportion of 2 to 8%, for example 3 to 8%, by weight of the composition, cetostearyl alcohol in a proportion of 3 to 6% by weight of the composition, and water. These proportions give a lotion, i.e. a composition which can be poured. If a cream is desired, the proportions of stearic acid and cetostearyl alcohol may be appropriately increased, e.g. up to about 10% by weight in each case.

Preferably, the compositions in accordance with the invention contain other ingredients. Thus, it is desirable to include a zinc supplement, e.g. a zinc soap such as zinc stearate, in the composition to promote healthy skin growth. An appropriate concentration is 0.5 to 2% by weight of the composition.

It is desirable to include a bactericide to protect against microbiological contamination. A cationic bactericide such as cetrimide is included as this has the added advantage that it is an emulsifier which promotes the homogeneity and stability of the composition. It may be present in a concentration of 0.5 to 2% by weight of the composition. Chlorocresol in a concentration of 0.05 to 0.2% by weight may be added for additional preservation.

To provide the treated area of skin with further protection, it may be advantageous to include in the compositions of the invention an oil soluble polysiloxane such as that sold under the trade mark Dimethicone ®. Such a silicone forms non-volatile barrier film on the treated area of skin which assists in keeping out infection and reducing local evaporation and thus drying out of the skin. Further, since areas prone to pressure sores are frequently areas subject to friction, it may be advantageous to include a lubricant in the new compositions. Appropriate lubricants are lower alkyl esters of long chain fatty acids of, for example, l2 to l8 carbon atoms, such as isopropyl myristate. The silicone and the lubricant may be present in a concentration of 5 to l0% and 5 to l5% by weight of the composition respectively. The upper limit on the lubricant concentration may be l0% by weight.

2

Other conventional ingredients in compositions for topical application may also be included, e.g. glycerol in a concentration of 0.5 to 5% by weight of the composition.

The new compositions may be made by dissolving the water-soluble ingredients, e.g. the bactericide, in purified water, which has, if necessary, previously been heated to remove dissolved air at a temperature of preferably 80-100°C. The oily constituents of the vehicle are separately heated, preferably to 80-100°C, and the oleophilic constituents of the composition are thoroughly mixed in. The aqueous and oily constituents are then combined and homogenized, preferably at 50-60°C. The nicotinate ester is then added, preferably at 40-50°C, and finally the composition is filled into sterile containers.

The effect of the compositions in accordance with the invention in promoting local blood flow has been demonstrated using a laser Doppler flowmeter (see "Laser Doppler Measurement of Cutaneous Blood Flow" G.A. Holloway and D.W. Watkins, Journal of Investigative Dermatology 69, 306-309, (1977). This flowmeter is used to shine laser-produced red light into the area of skin under study before and after treatment. Any increase in the rate of flow on the red blood cells in the illuminated areas produces a difference in the Doppler effect shown by the reflected light, since reflections from essentially stationary surfaces can be differentiated by this means from reflections from moving red blood cells. The blood cell flux is then given by the product of the number of red blood cells found to be moving in the irradiated volume of skin and adjacent tissue and the mean red blood cell velocity. The results expressed by the flowmeter indicate the relative change of the blood cell flux over control conditions, i.e. before application of the composition.

The following table shows the results obtained using the composition of Example I below on ten subjects:

| Subject No. | Sex | Age | Site[1] | Conc.[2] | Erythema[3] | Area[4] ($cm^2$) |
|---|---|---|---|---|---|---|
| 1 | F | 30 | A | V | 0 | 0 |
| | | | | 0.025 | 0 | 0 |
| | | | | 0.05 | 0.5 | 80.90 |
| | | | | 0.10 | 1 | 152.26 |
| 2 | F | 38 | A | V | 0 | 0 |
| | | | | 0.025 | 0.5 | 124.05 |
| | | | | 0.05 | 1 | 126.32 |
| | | | | 0.10 | 2 | 119.99 |
| 3 | M | 20 | A | V | 0 | 0 |
| | | | | 0.025 | 0 | 0 |
| | | | | 0.05 | 0 | 32.17 |
| | | | | 0.10 | 2 | 103.06 |
| 4 | F | 37 | A | V | 0 | 0 |
| | | | | 0.025 | 0 | 0 |
| | | | | 0.05 | 0 | 0 |
| | | | | 0.10 | 0 | 0 |
| 5 | M | 51 | A | V | 0 | 0 |
| | | | | 0.025 | 0 | 0 |
| | | | | 0.05 | 0.5 | 89.59 |
| | | | | 0.10 | 2 | 82.80 |
| 6 | M | 40 | B | V | 0 | 0 |
| | | | | 0.025 | 0 | 5.00 |
| | | | | 0.05 | 1 | 91.86 |
| | | | | 0.10 | 2 | 246.74 |
| 7 | F | 19 | B | V | 0 | 0 |
| | | | | 0.025 | 0 | 0 |
| | | | | 0.05 | 0.5 | 22.28 |
| | | | | 0.10 | 1 | 119.97 |
| 8 | M | 35 | B | V | 0 | 0 |
| | | | | 0.025 | 0 | 0 |
| | | | | 0.05 | 0 | 4.29 |
| | | | | 0.10 | 0.5 | 0 |
| 9 | F | 28 | B | V | 0 | 0 |
| | | | | 0.025 | 0 | 0 |
| | | | | 0.05 | 0 | 0 |
| | | | | 0.10 | 0.5 | 5.24 |
| 10 | F | 38 | B | V | 0 | 0 |
| | | | | 0.025 | 1 | 51.59 |
| | | | | 0.05 | 1 | 64.73 |
| | | | | 0.10 | 3 | 199.76 |

1. A = arms, B = buttocks
2. % hexyl nicotinate or V = Venicle only
3. Score 0–3 (0 = No erythema, 1 = slight erythema, 2 = moderate erythema, 3 = severe erythema)
4. Adjusted to a sensitivity of 1, area under the curve integrates the total change occurring

4

**Summary**

| Concentra-tion | Erythema | Area (cm$^2$) | No. measurable responses | Time to response (min ± S.D.) | | |
|---|---|---|---|---|---|---|
| | | | | Initial | Peak | Return to zero |
| V | 0 | 0 | 0 | — | — | — |
| 0.025 | 0.15 | 18.05 | 2 | 5.5 ± 0.7 | 22.5 ± 6.4 | 78.5 ± 16.3 |
| 0.05 | 0.45 | 51.21 | 8 | 10.5 ± 5.0 | 27.9 ± 12.3 | 75.8 ± 19.2 |
| 0.10 | 1.40 | 102.98 | 8 | 7.8 ± 2.3 | 20.8 ± 6.4 | 83.3 ± 17.7 |

Using an erythema scale of 0 to 3, an erythema value of up to 0.5 (which is barely detectable) is considered acceptable. The figures for "area" given in the third column are obtained by graphical integration of a graph of blood cell flux against time obtained using the laser Doppler flowmeter. Any figure in this column greater than about 20 may be regarded as indicative of a useful improvement in local blood flow in the treated area. The table clearly shows that such an improvement can readily be obtained with a degree of erythema of 0.5 or less. Thus the compositions in accordance with the invention make it possible to obtain a useful increase in local blood flow without appreciable erythema. The compositions of the present invention are thus useful in preventing pressure sores where is it desired to stimulate local blood flow without obvious erythema.

An increased local blood flow following application of the composition in accordance with the present invention is observable for typically 10 to 90 minutes after the application. Since continuous improved blood flow is not required for prevention of pressure sores, application twice or three times a day is sufficient.

The following Examples illustrate compositions according to the invention.

Example I

A composition was formulated containing (% by weight):

| | |
|---|---|
| Hexyl Nicotinate | 0.05 |
| Zinc Stearate | 1.00 |
| Stearic Acid | 5.00 |
| Cetostearyl Alcohol | 4.00 |
| Cetrimide | 1.00 |
| Dimethicone® 350 | 7.50 |
| Isopropyl Myristate | 7.50 |
| Glycerol | 2.00 |
| Chlorocresol | 0.10 |
| Purified Water | 71.85 |
| | 100.00 |

This composition 0.05% hexyl nicotinate, the most preferred amount. The other compositions used in the test contained the amounts of hexyl nicotinate specified in the table given above, an appropriate adjustment being made in the amount of water.

Example 2

A composition was formulated containing (% by weight).

| Hexyl Nicotinate | 0.05 |
|---|---|
| Zinc Stearate | 1.00 |
| Stearic Acid | 3.00 |
| Cetostearyl Alcohol | 4.00 |
| Cetrimide | 1.00 |
| Dimethicone 350 | 7.50 |
| Isopropyl Myristate | 12.50 |
| Glycerol | 2.00 |
| Chlorocresol | 0.10 |
| Purified Water | 68.85 |
| | 100.00 |

This composition has the advantage of being less viscous than that of Example I.

The compositions of Examples I and 2 (and other compositions of the invention may be manufactured as follows:

(a) The purified water is heated preferably to 80-I00°C to remove dissolved air. The cetrimide, glycerol and chlorocresol (i.e. the water-soluble ingredients) are added with stirring until completely dissolved.

(b) The aqueous solution obtained is allowed to cool to 50-60°C, preferably 55°C.

(c) The stearic acid, cetostearyl alcohol, Dimethicone® 350, isopropyl myristate and zinc stearate (i.e. the oily vehicle ingredients and the other oleophilic ingredients) are mixed together by warming to 60-I00°C, preferably 60°C and allowed to cool to 50-60°C, preferably 55°C.

(d) The aqueous phase from step (b) is added to the oily phase from step (c) and the mixture emulsified by homogenisation to produce a smooth lotion. The emulsion is then allowed to cool.

(e) The hexyl nicotinate is added to the product step (d) at 40-50°C, preferably 50°C, and mixed in thoroughly.

(f) When the lotion obtained has cooled to 37-43°C, preferably 40°C, it is filled into 350 ml round high density polyethylene bottles or other suitable sterile containers, preferably sealed with a pump dispenser principally constructed of high density polyethylene or other suitable material.

## Claims for the Contracting Strates BE, FR, DE, IT, LU, NL, SE, CH, LI

1. A pharmaceutical composition for topical application comprising hexyl nicotinate in a concentration from 0.025 to 0.075% by weight based on the weight of the composition, and an appropriate vehicle.

2. A composition according to claim 1 which also contains a zinc supplement.

3. A composition according to claim 2 in which the zinc supplement is present in a concentration of 0.5 to 2% by weight based on the weight of the composition.

4. A composition according to any of claims 1 to 3 in which the vehicle comprises stearic acid, cetostearyl alcohol and water.

5. A composition according to claim 4 in which the vehicle comprises 2 to 10% by weight of stearic acid and 2 to 10% by weight of cetostearyl alcohol, based on the total weight of the composition, the balance of the composition being water.

6. A composition according to any one of claims 1 to 5 which also contains one or more of a cationic bactericide, an oil soluble polysiloxane, and a lubricant.

7. A composition according to claim 6 which contains 0.5 to 2% of cetrimide as cationic bactericide, 5 to 10% of an oil-soluble polysiloxane, and 5 to 15% of isopropyl myristate, the said percentages being by weight based on the total weight of the composition.

8. Use of hexyl nicotinate in the preparation of a pharmaceutical composition containing 0.025 to 0.075% by weight based on the weight of the composition of hexyl nicotinate, for use in therapy to promote peripheral blood flow without causing observable erythema.

## Claims for the Contracting State AT

1. Process for the preparation of a pharmaceutical composition for topical application which comprises incorporating hexyl nicotinate in a concentration from 0.025 to 0.075% by weight based on the weight of the composition, in an appropriate vehicle.

2. Process according to claim 1 in which a zinc supplement is also incorporated in the composition.

3. Process according to claim 2 in which the zinc supplement is incorporated in a concentration of 0.5 to 2% by weight based on the weight of the composition.

4. Process according to any of claims 1 to 3 in which the vehicle used comprises stearic acid, cetosteryl alcohol and water.

5. A process according to claim 4 in which the vehicle used comprises 2 to 10% by weight of stearic acid and 2 to 10% by weight of cetostearyl alcohol, based on the total weight of the composition, the balance of the composition being water.

6. A process according to any one of claims 1 to 5 in which one or more of a cationic bactericide, an oil-soluble polysiloxane, and a lubricant, are also incorporated in the composition.

7. A process according to claim 6 in which 0.5 to 2% of cetrimide as cationic bactericide, 5 to 10% of an oil-soluble polysiloxane, and 5 to 15% of isopropyl myristate, the said percentages being by weight based on the total weight of the composition, are incorporated in the composition.

## Patentansprüche für die Vertragsstaaten BE , FR, DE, IT, LU, NL, SE, CH, LI

1. Pharmazeutische Zusammensetzung zur örtlichen Behandlung, welche 0,025 bis 0,075 Gew.-% Nikotinsäureethylester, bezogen auf das Gesamtgewicht der Zusammensetzung sowie einen geeigneten Träger enthält.

2. Zusammensetzung nach Anspruch 1, welche zusätzlich noch einen Zink-Zusatz enthält.

3. Zusammensetzung nach Anspruch 2, in welcher der Zink-Zusatz in einer Konzentration von 0,5 bis 2 Gew.-%, bezogen auf das Gesamtgewicht der Zusammensetzung vorhanden ist.

4. Zusammensetzung nach einem der Ansprüche 1 bis 3, in welcher der verwendete Träger die Stoffe Stearinsäure, Cetostearylalkohol und Wasser enthält.

5. Zusammensetzung nach Anspruch 4, in welcher der verwendete Träger 2 bis 10 Gew.-% Stearinsäure und 2 bis 10 Gew.-% Cetostearylalkohol, jeweils bezogen auf das Gewicht der Gesamt-Zusammensetzung, Rest Wasser, enthält.

6. Zusammensetzung nach einem der Ansprüche 1 bis 5, welche noch zusätzlich ein kationisches Bakterizid, ein öl-lösliches Polysiloxan und ein Gleitmittel einzeln oder zu mehreren enthält.

7. Zusammensetzung nach Anspruch 6, welche 0,5 bis 2 Gew.-% Cetrimid als kationisches Bakterizid, 5–10 Gew.-% eines öl-löslichen Polysiloxans und 5 bis 15 Gew.-% i-Propyl-Myristat, jeweils bezogen auf das Gesamtgewicht der Zusammensetzung enthält.

8. Verwendung von Nikotinsäurehexylester zur Herstellung einer pharmazeutischen Zusammensetzung mit 0,025 bis 0,075 Gew.-% Nikotinsäurehexylester bezogen auf das Gesamtgewicht der Zusammensetzung zur Anwendung bei der therapeutischen Förderung der Oberflächendurchblutung ohne beobachtbare Reizung.

## Patentansprüche für den Vertragsstaat AT

1. Verfahren zum Herstellen an einer pharmazeutischen Zusammensetzung zur örtlichen Behandlung, bei welchem Nikotinsäurehexylester in einer Konzentration von 0,025 bis 0,075 Gew.-%, bezogen auf das Gewicht der Gesamtzusammensetzung, einem geeigneten Träger zugefügt wird.

2. Verfahren nach Anspruch 1, bei welchem in die Zusammensetzung zusätzlich noch ein Zink-Zusatz eingearbeitet wird.

3. Verfahren nach Anspruch 2, bei welchem der Zink-Zusatz in einer Konzentration von 0,5 bis 2 Gew.-%, bezogen auf das Gesamtgewicht der Zusammensetzung eingearbeitet wird.

4. Verfahren nach einem der Ansprüche 1 bis 3, bei welchem der verwendete Träger die Stoffe Stearinsäure, Cetostearylalkohol und Wasser enthält.

5. Verfahren nach Anspruch 4, bei welchem der verwendete Träger 2 bis 10 Gew.-% Stearinsäure und 2 bis 10 Gew.-% Cetostearylalkohol, jeweils bezogen auf das Gewicht der Gesamt-Zusammensetzung, Rest Wasser, enthält.

6. Verfahren nach einem der Ansprüche 1 bis 5, bei welchem der Zusammensetzung noch ein kationisches Bakterizid, ein öl-lösliches Polysiloxan und ein Gleitmittel einzeln oder zu mehreren zugesetzt wird.

7. Verfahren nach Anspruch 6, bei welchem in die Zusammensetzung 0,5 bis 2 Gew.-% Cetrimid als kationisches Bakterizid, 5–10 Gew.-% öl-lösliches Polysiloxan und 5 bis 15 Gew.-% i-Propyl-Myristat jeweils bezogen auf das Gesamtgewicht der Zusammensetzung eingearbeitet werden.

## Revendications pour les Etats Contractants BE, FR, DE, IT, LU, NL, SE, CH, LI

1. Composition pharmaceutique pour application topique comprenant du nicotinate d'hexyle à une concentration comprise entre 0,025 et 0,075% en poids par rapport au poids total de la composition, et un véhicule approprié.

2. Composition selon la revendication 1, qui contient également un additif à base de zinc.

3. Composition selon la revendication 2, dans laquelle l'additif à base de zinc est présent à une concentration comprise entre 0,5 et 2% en poids par rapport au poids de la composition.

4. Composition selon l'une des revendications 1 à 3, dans laquelle le véhicule comprend de l'acide stéarique, de l'alcool cétostéarylique et de l'eau.

5. Composition selon la revendication 4, dans laquelle le véhicule comprend 2 à 10% en poids d'acide stéarique et 2 à 10% en poids d'alcool cétostéarylique, par rapport au poids total de la composition, le reste de la composition étant constitué par de l'eau.

6. Composition selon l'une des revendications 1 à 5, qui contient également un ou plusieurs produit(s) choisi(s) parmi un bactéricide cationique, un polysiloxane soluble dans l'huile et un agent lubrifiant.

7. Composition selon la revendication 6, qui contient 0,5 à 2% de cétrimide comme bactéricide cationique, 5 à 10% de polysiloxane soluble dans l'huile et 5 à 15% de myristate d'isopropyle, lesdits pourcentages étant en poids par rapport au poids total de la composition.

8. Utilisation de nicotinate d'hexyle dans la préparation d'une composition pharmaceutique contenant 0,025% à 0,075% de nicotinate d'hexyle en poids par rapport au poids de la composition de nicotinate d'hexyle, pour une utilisation thérapeutique augmentant la circulation sanguine périphérique sans provoquer d'érythème observable.

### Revendications pour l'Etat Contractant AT

1. Procédé de préparation d'une composition pharmaceutique pour application topique, qui comprend l'incorporation de nicotinate d'hexyle à une concentration comprise entre 0,025 et 0,075% en poids par rapport au poids de la composition, dans un véhicule approprié.

2. Procédé selon la revendication 1, dans lequel on introduit également dans la composition un additif à base de zinc.

3. Procédé selon la revendication 2, dans lequel l'additif à base de zinc est incorporé à une concentration comprise entre 0,5 et 2% en poids par rapport au poids de la composition.

4. Procédé selon l'une des revendications 1 à 3, dans lequel le véhicule utilisé comprend de l'acide stéarique, de l'alcool cétostéarylique et de l'eau.

5. Procédé selon la revendication 4, dans lequel le véhicule utilisé contient 2 à 10% en poids d'acide stéarique et 2 à 10% en poids d'alcool cétostéarylique, par rapport au poids total de la composition, le reste de la composition étant de l'eau.

6. Procédé selon l'une des revendications 1 à 5, dans lequel on incorpore également dans la composition un ou plusieurs produit(s) choisi(s) parmi un bactéricide cationique, un polysiloxane soluble dans l'huile et un agent lubrifiant.

7. Procédé selon la revendication 6, dans lequel on incorpore dans la composition 0,5 à 2% de cétrimide comme bactéricide cationique, 5 à 10% de polysiloxane soluble dans l'huile, et 5 à 15% de myristate d'isopropyle, lesdits pourcentages étant en poids par rapport au poids total de la composition.